# EUROPEAN PATENT SPECIFICATION

(11) **EP 0 889 902 B1**
(45) Date of publication and mention of the grant of the patent: **20.06.2001**
(21) Application number: 96910013.0
(22) Date of filing: 25.03.1996
(51) Int. Cl.: C07K 14/47, A61K 38/17, A23L 3/3463

(54) **ANTIBIOTIC PEPTIDES FROM BOVINE MILK**
ANTIBIOTISCHE PEPTIDE AUS DER KUHMILCH
PEPTIDES ANTIBIOTIQUES PROVENANT DU LAIT DE VACHE

(43) Date of publication of application: 13.01.1999
(73) Proprietor: Pharis Biotec GmbH, 30625 Hannover (DE); Forssmann, Wolf-Georg, Prof. Dr. med., 30625 Hannover (DE)
(72) Inventor: FORSSMANN, Wolf-Georg, D-30625 Hannover (DE); ZUCHT, Hans-Dieter, N-sächs.Inst.Peptid-Forsch.IPF, 30539 Hannover (DE); RAIDA, Manfred, 30163 Hannover (DE); ADERMANN, Knut, Niedersächs.Inst.Peptid-Forsch.IPF, 30625 Hannover (DE); MÄGERT, Hans-Jürgen, N-sächs.Ins.Peptid-Forsch.IPF, 30625 Hannover (DE)
(74) Representative: Meyers, Hans-Wilhelm, Dr.Dipl.-Chem.
(86) International application number: EP9601296
(87) International publication number: WO9735877

(56) References cited:
- DE-A- 4 444 753
- FEBS LETTERS 372 (2-3). 1995. 185-188, XP000524602 ZUCHT H-D ET AL: "Casocidin -I: A casein -alpha-s2 derived peptide exhibits antibacterial activity." cited in the application
- EXPERIMENTAL BIOLOGY 96, PART II, WASHINGTON, D.C., USA, APRIL 14-17, 1996. FASEB JOURNAL 10 (3). 1996. A796, XP002020116 ZUCHT H D ET AL: "Antimicrobial properties of casocidin -I: A peptide processed from casein -alpha-s2."
- CHEMICAL ABSTRACTS, vol. 124, no. 3, 15 January 1996 Columbus, Ohio, US; abstract no. 28228, AKAZOME, YOKO ET AL: "Characteristics and application of milk protein hydrolyzates" page 840; XP002020117 & RAKUNO KAGAKU, SHOKUHIN NO KENKYU (1995), 44(4), A129-A138 , 1995,

## Description

The present invention is concerned with peptides having the amino acid according to claim 1, fragments of the peptides obtainable by proteolytic cleavage, a medicament comprising a peptide of the invention, a process for the manufacturing of peptides of the invention as well as methods of using the peptides of the invention.

Antimicrobial peptides are small peptide compounds which are capable to decrease the incidence of disease and could serve as modulators of a naturally occurring bacterial flora (Ganz T. et al., 1992, Med. Microbiol. Immunol. 181, 99 - 105; Eisenhauer P.B. et al., 1992, Infect. Immun. 60, 3556-3565; Oulette A. J. et al., 1992, FEBS Lett. 304, 146- 148; Jones D. E. and Bevins C. L., 1993, FEBS Lett. 315, 187- 192; Selstedt M.E. et al. (1992) J. Cell. Biol. 118, 929 - 936).

The fact that milk could influence micro-organisms is also well established (Wharton B. A. et al, 1994, Acta Paediatr. Jpn. 36, 579 - 584). Present factors thought to be responsible are lactoferrin (Bullen J.J. et al., 1972, Brit. J. Med. 1, 69 - 72; Baggiolini M. et al, 1970, J. Exp. Med. 13, 559 - 570), lysozyme (Fleming A., 1922, Proc. Roy. Soc., London, 93, 306 - 317, Jolles J. and Jolles P., 1968, Bull. Soc. Chim. Biol., Paris, 50, 2543 - 2551), lactoperoxidase (Cals M. M. et al., 1991, Eur. J. Biochem. 198, 733 - 739, Bullen J. J. et al., 1972, Brit. J. Med. 1, 69 - 72). Milk is a rich source of peptides mainly derived from proteolytic cleavage of proteins. Beyond a nutritive value several biological effects were described like immunomodulation, antithrombotic activities, opioid action or inhibition or mineral carriage (Meisel H. et al., 1989, Z. Ernährungswiss. 28, 267 - 278; Fiat A. M. and Jolles P., 1989, Mol. Cell. Biochem. 87, 5 - 30; Fiat A. M. et al., 1993, J. Dairy. Sci. 76, 301 - 310).

Casocidin-I is a peptide compound, a defined cleavage product from casein-alpha-s2, which is naturally occurring in bovine milk as it could be purified in authentic form (Zucht H.D., 1995, FEBS Lett. 372, 185 - 188). It has no counterpart in human milk since human milk does not contain any casein of the alpha-s2 type.

Surprisingly, the mild antibacterial effect found in milk could be enriched several orders of magnitude by the peptides of the invention. According to the invention a peptide with antibiotic effects show the amino acid sequence

H₂N-X₁-R-X₃-X₂-COOH (formula I)

wherein
X₁ is either zero or
X₁ and/or X₂ are a residue representing at least five amino acid residues (symbolized in the one letter amino acid code), preferably naturally occurring amino acids,
with the proviso that
X₁ and/or X₂ contain at least one basic amino acid residue immediately followed by a hydrophobic amino acid residue and X₁ and/or X₂ contain at least one glutamine residue, with the proviso
X₁ does not represent: KTKLTEEEKNRLNFLKKISQ
X₁ does not represent: KNRLNFLKKISQ
X₂ does not represent: RYQFALPQYLKTVYQHQK

Preferably, the distance of the hydrophobic residue and R should not exceed 7 - 10 amino acids.

It has now been found that these peptides could kill a broad range of microorganisms with a preference of microorganisms not naturally present in the normal intestinal flora. Surprisingly, the peptides of the invention show activity also against eukaryotic organisms such as fungi but do not effect other eukaryotic cells as for example HT 29 cells. The peptides of the invention are therapeutical tools for treating diseases caused by pathogenic eukaryots, for example, protists which are only present in the adult cattle intestine and that of man respectively.

In a preferred embodiment the peptide of the invention has a structure wherein
X₁ represents -Xₘ-K-B-Xₙ- wherein
X is any amino acid residue having at least one Q,
B is any hydrophobic amino acid residue;
m = 0 - 40, n = 0 - 8 and
X₂ is Q.

A further preferred embodiment of the peptide of the invention is a structure wherein
X₁ is zero or represents Q,
X₂ represents -Xₙ-K-B-Xₘ-,
X, B, m and n having the same meaning as defined above.

A further preferred embodiment of the present invention is a peptide having a structure wherein
X₁ represents -Xₘ-K-B-Xₙ- and
X₂ represents -Xₙ-K-B-Xₘ,
X,B,m, and n have the same meanings as defined above.

It is understood by the skilled person that not only amino naturally occurring can be used in effective peptides. Therefore, the amino acids in the chain forming the peptides can be modified in order to match valuable properties for respective use. For example, it is possible to use mixtures of D- and/or L-amino acids in the amino acid sequence of the peptides of the invention in order to modify the pharmaceutical effects.

Furthermore, there may be introduced modification in the side chains of the peptide backbone of the amino acid sequence. For example, it is possible to introduce sugar moieties at suitable positions, for example, asparagine. Glycosylation modifies affinity of the peptides or its solubility. It may also be useful to phophorylate amino acids of the peptide of the invention. It may also be useful to modified amino acid side chains in order to modify the hydrophobicity or hydrophilicity of the peptide. For example, if the peptide is used in a pharmaceutical composition which is designed for topical application it may be advantageous to modify the peptide that it more easily penetrates the skin in order to develop its activity also in deeper layers of the skin. On the other hand if only a surface treatment is needed the properties of the peptide could be modified in so far as it is not penetrating too far into the deeper layers of the skin.

A further modification may occur at the N-terminal or C-terminal end of the peptide. The N-terminal end may be modified with electrophilic agents. For example, the N-terminal amino group may be alkylated, acylated, cleaved off or protected. The N-terminal end may be protected with labile chemical groups which are cleaved off during the use of the peptide. The C-terminal end of the peptides can be modified with nucleophilic reagents in order to attack the carbonyl group of the carboxyl and the C-terminal end may be modified, for example by amidation, esterification and the like.

It is possible to obtain peptides by proteolytic cleavage of the peptides of the invention. Such fragments which fall into the scope of the invention must show antifungal activity after protease treatment. Suitable proteases for obtaining fragments from the peptides of the invention are endo-proteinases like trypsin, subtilisin, thermolysin and endo-proteinase LYS-C.

Preferably, the peptide of the invention consists of a domain -RYQ-. However, for example, a tetrapeptide with the motif QRYQ alone is not sufficient to gain antimicrobial properties.

A further preferred structure on both sides of this core motif -RYQ- is the occurrence of one or more basic residues immediately followed by a hydrophobic amino acid residue. The specificity of the peptides of the invention can be modulated by composition of the residues flanking the core region -RYQ- so that the detailed antimicrobial spectrum of the peptide of the invention could be fine tuned by means of modulating residues around the core motif.

Within the side chain one or more glutamine residues seem to be of some relevance since the preferred peptide casocidin-I (casocidin 1 - 39) contains high an amount of glutamine residues. On the other side the glutamine residues alone do not promote the antimicrobial effect as casein-alpha-s2-80-120 shows a very similar glutamine pattern but does not exhibit any antibacterial effects.

Specifically preferred peptides according to the inventions are those as follows:

As the skilled person understand also this very specific peptides can be modified according to the strategies outlined above discussing the general aspects of the invention.

According to the invention the process for the manufacturing of a peptide according to the invention comprises the steps of
- treating bovine milk with acetic acid and calcium sulfate,
- heating the mixture obtained
- removing the precipitate and isolating the supernatant,
- treating the supernatant with a cation-exchanger resin for binding of basic peptides, washing the resin optionally with a urea solution to remove non specifically bound material,
- isolating the resin having peptides bound to it,
- elution of peptides bound to the resin and further purification with chromatographic operations,
- optionally followed by fragmentation, e.g. by digestion with proteases.

The precise conditions are exemplified in the working examples of the specification herein below. The biological activity of the peptides of the invention show a broad range specificity against grampositive and gramnegative bacteria. They are also capable to inhibit the growth of fungi exemplified on Rhodotorula rubra. Intestinal cells were not killed. This shows that they are specificly effective against the pathogene but not against the host. Preferably, the activity is pronounced in low ion strength media in the absence of high amounts of calcium. It is preferred to administer the peptide of the invention in a hypotonic medium in the presence of calcium complexing agents such as citrate, phosphate, EGTA in order to facilitate its activity. The mechanism of action may be due to the membrane permeabilizing effect of the peptide depending on the dosage of the peptides of the invention.

The peptides of the invention can be used for formulating a medicament for treating diseases which are caused by bacteria or fungi. The medicament optionally contains further active compounds or carrier materials and or adjuvants. In a preferred embodiment of the present invention the medicament of the invention is formulated for topical administration or administration on mucosa such as the mucosa of mouth and vagina. Because of its antifungal potency the medicament of the present invention is preferably useable for treating fungal diseases and or treatment of diseases which are caused by eukaryotic organisms such as trichomonades, for example in vagina, or organisms such as filaria or plasmodium.

The peptide of the invention preferably casocidin-I can be administered in the form of an oral dosage unit together with fillers which are pharmaceutically acceptable. It can be used in liquid preparations or in a dry preparation with lyophilized peptide. The typical dosage would be in the range of 1 *µ*g/ml up to 250 *µ*g/ml of the medicament. Casocidin-I keeps its potency when lyophilized and can be stored at room temperature in this form. It allows long term storage with reconstitution with an appropriate low ion strength buffer, for example, 25 mOsm prior to use. A supplementation with pharmaceutically acceptable agents capable to complex calcium like citrate (about 5 - 250 mM), phosphate (5 - 150 mM) or EGTA (1 mM) support the antimicrobial effect of the drug.

If antimicrobial diseases of the skin have to be treated the formulation preferably is in the form of an ointment. The concentration range of the drug in such a formulation is comparable to the above mentioned range of 0,1 ng/ml to 1 mg/ml, preferably 1 *µ*g/ml to 250 *µ*g/ml. The dosis can be adjusted obeying the rules of the art as can readily understood by the skilled person.

The peptides of the invention can be used for therapy of diseases such as diarrhoea, diseases caused by a change of the microbiological properties or conditions in the intestine, the mucosa of the vagina or the mouth, diseases of the skin and diseases caused by fungi.

The peptide of the invention can be used as preservative of food or perishable goods or as adjuvant for fermentation processes.

### Figure 1

Purification of casocidin-I: (a) RP-C18 HPLC of the peptides eluted from the cation exchanger. The growth inhibitory activity was monitored with E. coli in the radial diffusion assay. (b) RP-C18 HPLC rechromatography of the most active fraction. (c) RP-C4 HPLC rechromatography of fraction 13. (d) CZE analysis of the purification product casocidin-I.

### Figure 2

Effect of casocidin-I on the CFU of E. coli BL21 depending on incubation time and dosage. The picture shows various plates with bacterial colonies.

### Figure 3

Dose response curves of casocidin-I with diverse microorganisms in the preincubation experiment (determination of CFU).

### Figure 4

Radial diffusion experiment, indicating the antimicrobial activity of casocidin-I.

### Figure 5

Release of intracellular enzymes beta-lactamase and beta-galactosidase indicating the effect of casocidin-I.

The purification of casocidin-I out of bovine milk is exemplified as follows.

Grade A bovine milk is to be used. In order to denature most of the high molecular weight proteins, the milk must be initially treated by boiling for 5 min and acidifying with 10% (v/v) acetic acid. Additionally, 1 g/l calcium chloride (CaCl₂* 2 H₂O) is added to precipitate calcium-dependent phosphoproteins. After centrifugation (15 min, 3,500 x g, and 4°C) the resulting supernatant is collected, diluted with 4 volumes of water and incubated with a strong cation exchange resin (Parcomer, 20µm, Biotek, Heidelberg, Germany).

The resin is washed with 5 M urea in 5 mM phosphate buffer (pH 3.0) and with an excess of water. To elute bound peptides the resin is treated with 1 M NaCl in 5 mM phosphate buffer (pH 3.0) at room temperature. The eluate is centrifuged (5 min, 1,000 x g, RT) to remove residual resin particles.

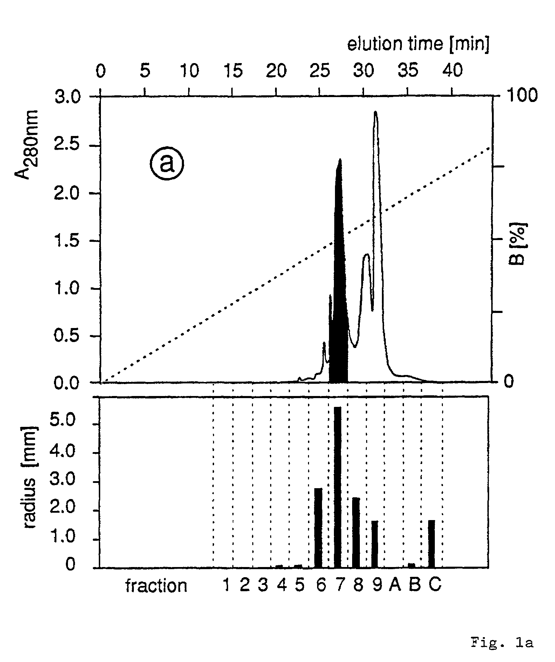

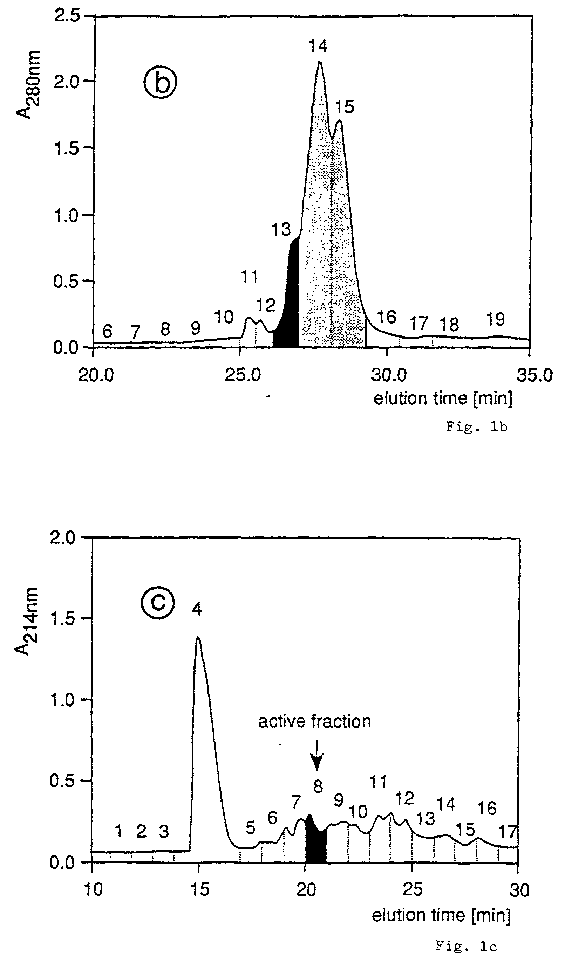

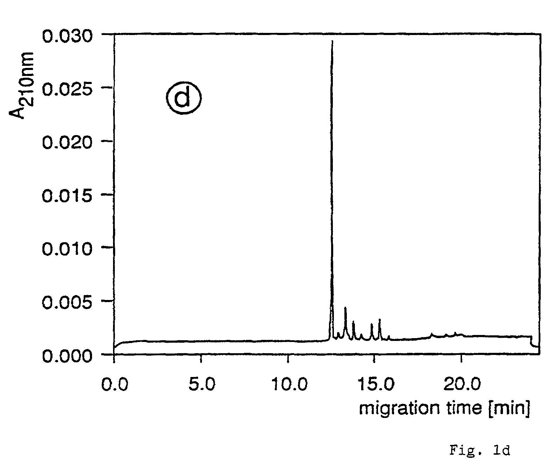

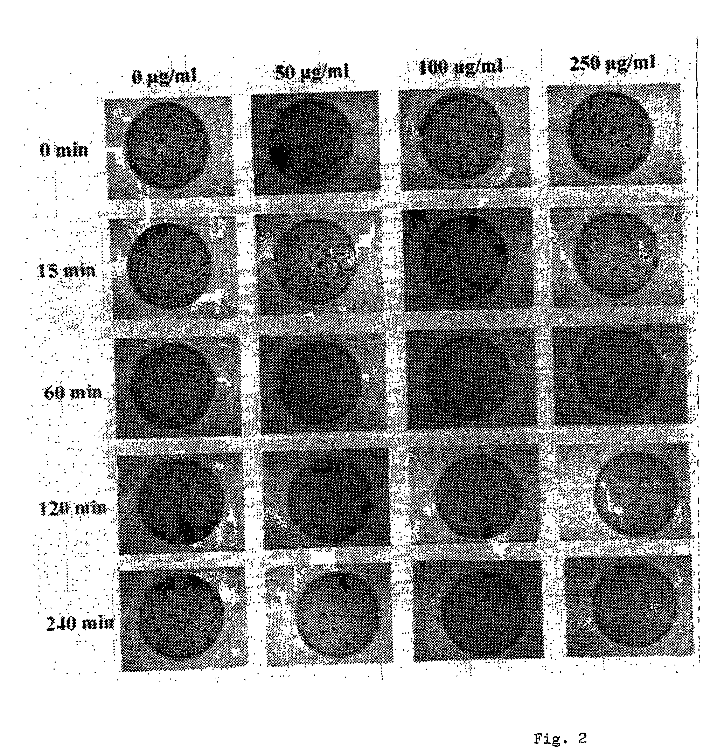

The HPLC purification steps were performed on Parcosil-C₁₈ (1 cm x 12.5 cm, 100 Å, 5 *µ*m) and Parcosil-C₄ (0.4 cm x 12.5 cm, 300 Å, 5 *µ* m) reversed phase columns (Biotek, Heidelberg, Germany). After equilibration with 0.1% trifluoroacetate (TFA), peptides are eluted by linearly increasing the amount of solvent B (acetonitrile with 0.1% TFA). For the first separation step a Parcosil C₁₈ RP column (1 cm x 12.5 cm, 100 Å, 5 *µ*m) is used with the following gradient: 0 - 50 min, 0 - 80% B. The bactericidal activity of an aliquot were monitored with the radial diffusion assay and the maximum activity was pooled. The second step of purification was performed with the same RP-C₁₈ column using a less steep gradient (gradient 2: 0 - 5 min, 0 - 15% B; 5 - 65 min, 15 - 80% B) (fig. 2). In the third step of purification, the antibiotic activity of fraction 13 could be recovered by RP-C₄ HPLC using the following gradient (0 - 5 min, 0 - 15% B; 5 - 65 min, 15 - 80% B). Fraction 8 contains casocidin-I with a relative high purity. The purification is illustrated in fig. 1 a - d.

The peptide analysis revealed the following structure of casocidin-I:

### SYNTHESIS OF CASOCIDIN-I

Chemical synthesis of peptides was carried out as described elsewhere (Atherton E. and Sheppard R.C., 1989, Solid phase peptide synthesis, IRL Press, Oxford; Jones, J., 1991, The chemical synthesis of peptides, Clarendon Press, Oxford). The purification was performed on a Vydac RP-C₁₈ column (MZ-Analysentechnik, Mainz, Germany, 10 *µ*m, 300 Å, 2 cm x 25 cm) with 0.06% TFA/acetonitrile/water at a flow rate of 10 ml/min. The purity was controlled by means of HPLC and mass spectrometry.

### ANTIMICROBIAL ASSAYS

### BACTERIA

E. coli Xll blue is a commercial strain distributed by Stratagene (USA). E. coli BL 21 (DE3) was a gift from A. Ebneth (Medical School Hannover, Dept. of Biophysics). Staphylococcus carnosus T300 was a gift from F. Goetz (Freiburg). Rhodotorula rubra, Staph. epidermidis is a clinical isolate from I. Zimmer and M. Weimann (laboratory of Clinical Medicine, Hannover). All other bacteria were purchased at Deutsche Sammlung von Mikroorganismen und Zellkulturen GmbH (DSM, Braunschweig, Germany). All bacteria were routinely cultured on LB-Broth (10 g/l caseinhydrolysate, 5 g/l yeast extract, 5 g/l NaCl, pH 7.4).

### PREINCUBATION EXPERIMENT

Exponentially grown bacteria with a density corresponding to an A₆₀₀ of 0.4 to 0.6, were initially diluted 1 : 10⁵ in 10 mM phosphate buffer (Na-P, pH 7.2) or phosphate buffered saline (PBS, pH 7.4) and incubated at 37°C for 120 min in Na-P or PBS supplemented with different concentrations of peptides. After this period the reaction mixtures were diluted 1 : 10 and 1 : 100 in the same buffer and plated on LB-Agar to determine the colony forming units (CFU). The plating were done in triplets. The colonies were counted with a colony counter (Darkfield, Quebec, Canada).

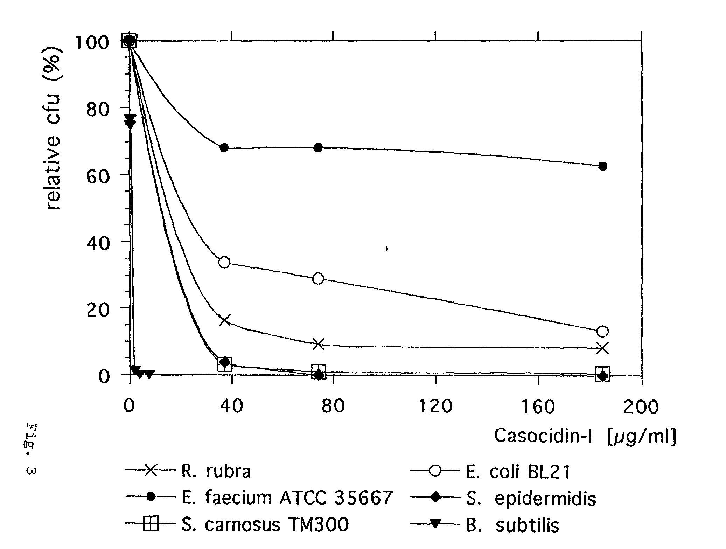

An example for the depletion of viable bacteria gives fig. 2 showing that a time dependent killing of the bacteria during incubation occurs. Further results of preincubation experiments are summarized in fig. 3. The interpolation of the date gives the following order of sensitivity of the used strains B. subtilis > S. carnosus = S. epidermidis > Rhodotorula >> E. coli > Enterococcus faecium ATCC 35667. The concentration necessary to reduce the colony forming units (CFU) to 50% of the initial CFU ranges from 180 *µ*g/ml (E. faecium) over 20 *µ*g/ml (E. coli, S. carnosus, R. rubra) to 1 *µ*g/ml for B. subtilis indicating a potent inhibitory effect of the used peptide.

### RADIAL DIFFUSION EXPERIMENT

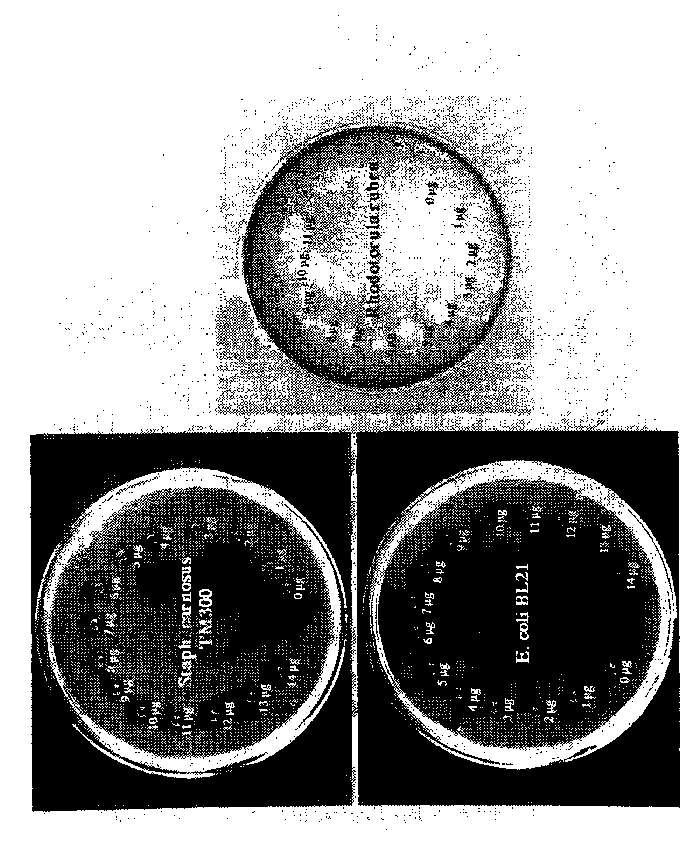

The radial diffusion assay performed as described in (Lehrer R.I. et al., 1991, J. Immun. Meth. 137, 167 - 173) with slight modifications. Bacteria were grown to a final absorbance at 580 nm of 0.2 (appr. 2 x 10¹⁰/ml). The agarose layer contained 30 mg/100 ml tryptic soy broth (TSB, Sigma chemicals, Germany) in 10 mM sodiumphosphate 10 mM pH 7.2 with 0.02% Tween® 20 and 0.6% agarose without EEO (Serva, Heidelberg, Germany). One ml of the bacteria suspension was added to 50 ml agarose at 50°C and the plates were poured immediately. Afterwards the plates were incubated at 4°C to harden the agarose. The bacteria were incubated for 10 hours at 37°C with the peptide substrate applied into holes of 3 mm diameter in the agarose layer. In some cases the plates were first fixed with 0.1% glutardialdehyde and afterwards stained with Giemsa stain (Merck, Darmstadt, Germany) diluted in 10 mM Tris-HCl pH 7.2 and washed with an excess of the same Tris Buffer. Using Rhodotorula rubra the medium contained 0.8% low melting point agarose (Sigma, Germany), 0.5 g/ 100 ml TSB and 20 mM TRIS-HCl pH 7.5. The yeast was added after an equilibration of the medium at 37°C and poured into the plates. The incubation was performed for 10 h at 30°C. The inhibitory influence of casocidin-I shows fig. 4.

### INFLUENCE OF MEDIUM STRENGTH AND CALCIUM IONS

To determine the minimal inhibitory concentration of casocidin-I under the influence of the medium strength or in the presence of bivalent cations, a dilution assay was performed in 96-well cell culture clusters. After diluting the peptide in 50 *µ*l medium (tryptic soy broth, Sigma, Germany) with adjusted pH 7.2, ion concentrations or medium strength on one plate, the same amount of medium with appr. 2 - 4 * 10³ bacteria in 50 µl medium were added to the diluted samples. The incubations were done overnight at 37°C. The minimal inhibitory concentration was manually read using a microscope.

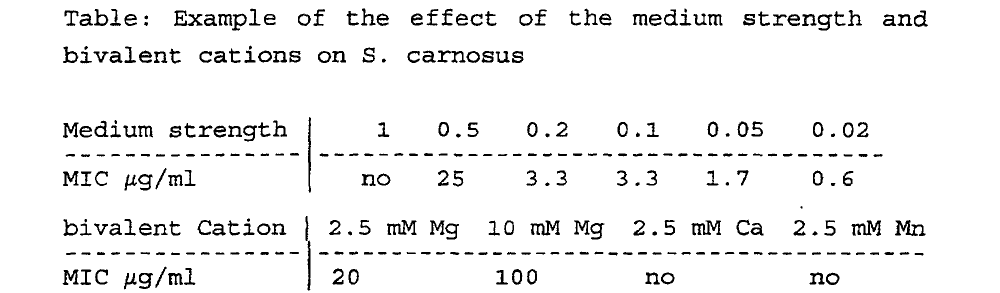

### Influence of casocidin on cell lines

HT 29 (human colon carcinoma) cells were grown as monolayers at 37°C in RPMI 1640 medium (Gibco, Germany) containing 2 mM L-glutamine, 100 U/ml penicillin, 0.1 mg/ml streptomycin and 10% heat inactivated fetal calf serum in 5% CO₂ on 24-well tissue culture clusters (Nunc, Germany) by loading of a suspension of 5 * 10⁵ cells/ml. To determine the cytotoxic effect of casocidin-I, HT 29 cells propagated in tissue culture clusters were incubated with different concentrations of casocidin-I (from 1 *µ*g/ml to 100 µg/ml) for up to 24 h at 37°. The experiments show no cytotoxic effects to this intestinal cell line.

### DETERMINATION OF THE MEMBRANE PERMEABILIZATION

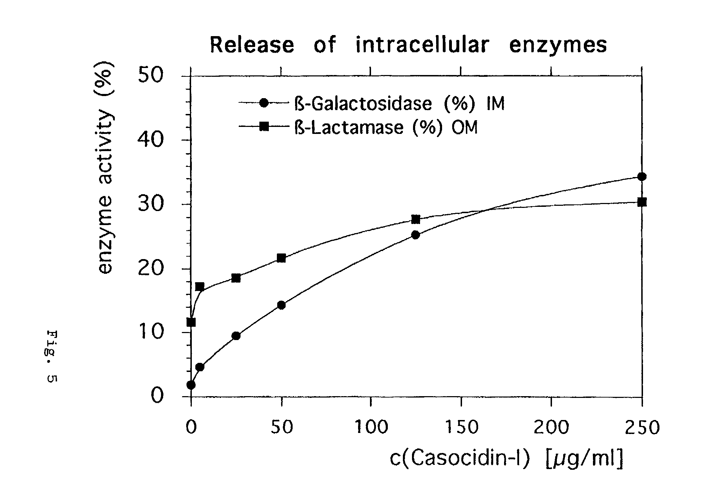

To determine the mechanism of action of casocidin-I which is a membrane permeabilizing effect we determined the release of intracellular enzymes beta-galactosidase and beta-lactamase from E. coli transformed with the plasmid pUC18 (fig. 5). The beta-galactosidase indicated the degree of the permeabilization of the inner membrane (IM) of E. coli. The permeabilization of the outer membrane was monitored by determination of released beta-Lactamase (plasmid coded) mainly present in the periplasmatic space.

To measure the relative release of beta-galactosidase a suspension of E. coli grown overnight in LB medium was washed once in phosphate buffer (10 mM, pH 7.0). Afterwards the bacteria were resuspended to a final density of app. 0.5 A₆₀₀. Peptide fractions were lyophilized in 1.5 ml tubes in a speed vac concentrator and solved in 40 *µ*l phosphate buffer. To the peptide solution a volume of 200 *µ*l bacterial suspension was added and the incubation was performed at 37°C for 30 min. As a control, to determine the maximum of the enzyme release, bacteria were disrupted by ultrasonic treatment (about 100 joules in 10 sec). The samples were afterwards centrifuged for 5 min at 15,000 x g at 4°C and the supernatant immediately removed from the sediment. To detect the beta-galactosidase activity an amount of 40 *µ*l lysate was mixed with 460 *µ*l of the detection reagent (60 mM Na₂HPO₄, 400 mM NaH₂PO₄, 10 mM KCl, 1 mM MgSO₄, 50 mM betamercaptoethanol, 0.89 mg/ml o-nitro phenyl galactoside ONPG) and incubated at 37°C for 45 min. The absorbance of the samples at 405 nm was measured and the increase of each absorbance was normalized to the sonified sample assuming enzymatic release of 100%.

To detect the release of beta-lactamase the supernatants of E. coli were prepared as described for the beta-galactosidase. As washing buffer PBS was used. To monitor the beta-lactamase activity an amount of 50 µl. supernatant was mixed with 50 *µ*M PADAC (Calbiochem, USA) in 10 mM Na-P pH 7.2. The samples were incubated for 45 min at 37°C in the dark and the absorbance was measured at a wavelength of 700 nm and the normalization was done with the results gained with sonified samples.

### PEPTIDE ANALYSIS

Amino acid sequence determinations were carried out automatically on an Applied Biosystems 473 A gas phase sequencer (Applied Biosystems Div. of Perkin Elmer, Weiterstadt, Germany). Capillary zone electrophoresis (CZE) was performed with a 50 cm uncoated capillary of fused silica on the CZE system model P/ACE 2100 (Beckmann, München, Germany) . The running buffer used was 100 mM NaH₂PO₄, pH 2.5 containing 0.02% hydroxypropyl methyl cellulose. After injection of 60 nl sample, the separation was carried out with a constant current of 120 *µ*A. Mass spectrometric analysis (MS) was performed on a triple-stage quadrupole electrospray mass spectrometer Sciex API III (Perkin Elmer, Ueberlingen, Germany) equipped with an articulated ion-spray source operation at atmospheric pressure. The samples were diluted in 50% acetonitrile, 0.2% acetic acid prior to injection. Mass spectra were recorded in positive ion mode.

## Claims

1. A peptide having the amino acid sequence
H₂N-X₁-R-X₂-COOH (formula I)
wherein
X₁ is either zero or
X₁ and/or X₂ are a residue representing at least five amino acid residues (symbolized in the one letter amino acid code), preferably naturally occurring amino acids,
with the proviso that
X₁ and/or X₂ contain at least one basic amino acid residue immediately followed by a hydrophobic amino acid residue and X₁ and/or X₂ contain at least one glutamine residue
with the proviso that
X₁ does not represent: KTKLTEEEKNRLNFLKKISQ
X₁ does not represent: KNRLNFLKKISQ
X₂ does not represent: RYQFALPQYLKTVYQHQK

2. The peptide according to claim 1 wherein
X₁ represents -Xₘ-K-B-Xₙ- wherein
X is any amino acid residue having at least one Q,
B is any hydrophobic amino acid residue;
m = 0 - 40, n = 0 - 8 and
X₂ is Q.

3. The peptide according to claim 1 wherein
X₁ is zero or represents Q,
X₂ represents -Xₙ-K-B-Xₘ-,
X, B, m and n having the same meaning as defined in claim 2.

4. The peptide according to claim 1 wherein
X₁ represents -Xₘ-K-B-Xₙ- and
X₂ represents -Xₙ-K-B-Xₘ,
X,B,m, and n have the same meanings as defined in claim 2.

5. The peptide according to any one of claims 1 - 4, having D- and/or L-amino acids.

6. The peptide according to anyone of the claims 1 - 5, having the structures

7. The peptide according to anyone of the claims 1 - 6 being chemically modified in the amino acid side chains and/or the amino group of the N-terminal end has been modified with electrophilic reagents and/or the carboxyl moiety of the C - terminal end of the peptide has been modified with nucleophilic reagents.

8. Fragments of the peptides according to anyone of the claims 1 - 7, obtainable by proteolytic cleavage with proteases, which fragments show antifungal and/or antibiotic effects.

9. A medicament comprising at least one of the peptides according to any one of the claims 1 - 8 in an effective amount for treating diseases, optionally in a mixture with further active compounds and or carrier materials or adjuvants.

10. The medicament of claim 9 formulated for topical administration or administration on mucosa.

11. The medicament of claim 9 for treating of fungal diseases, and/or treatment of diseases caused by eukaryotic organisms such as trichomonades in vagina, or organisms as filaria or plasmodium.

12. Method of using a peptide according to any one of the claims 1 - 8 as preservative of food or perishable goods or as adjuvant for fermentation processes.

13. Method of using a peptide according to any one of the claims 1 - 8 for the manufacturing of a medicament according to claims 9 - 11.

## Patentansprüche

1. Peptid mit der Aminosäuresequenz
H₂N-X₁-R-X₂-COOH (Formel I),
wobei
X₁ entweder Null ist; oder
X₁ und/oder X₂ ein Rest sind, der wenigstens fünf Aminosäurereste (symbolisiert im Ein-Buchstaben-Aminosäurecode), vorzugsweise natürlich vorkommende Aminosäuren, darstellt;
mit der Maßgabe, dass
X₁ und/oder X₂ wenigstens einen basischen Aminosäurerest enthalten, auf den unmittelbar ein hydrophober Aminosäurerest folgt, und X₁ und/oder X₂ wenigstens einen Glutaminrest enthalten;
mit der Maßgabe, dass
X₁ nicht **KTKLTEEEKNRLNFLKKISQ** darstellt;
X₁ nicht **KNRLNFLKKISQ** darstellt;
X₂ nicht **RYQFALPQYLKTVYQHQK** darstellt.

2. Peptid gemäß Anspruch 1, wobei
X₁ folgendes darstellt: -Xₘ-K-B-Xₙ-, wobei
X irgendein Aminosäurerest ist, der wenigstens ein Q aufweist;
B irgendein hydrophober Aminosäurerest ist;
m = 0-40 ist, n = 0-8 ist; und
X₂ Q ist.

3. Peptid gemäß Anspruch 1, wobei
X₁ Null ist oder Q darstellt;
X₂ folgendes darstellt: -Xₙ-K-B-Xₘ-;
X, B, m und n dasselbe wie in Anspruch 2 bedeuten.

4. Peptid gemäß Anspruch 1, wobei
X₁ folgendes darstellt: -Xₘ-K-B-Xₙ-; und
X₂ folgendes darstellt: -Xₙ-K-B-Xₘ-;
X, B, m und n dasselbe wie in Anspruch 2 bedeuten.

5. Peptid gemäß einem der Ansprüche 1-4, das D- und/oder L-Aminosäuren aufweist.

6. Peptid gemäß einem der Ansprüche 1-5 mit der Struktur oder

7. Peptid gemäß einem der Ansprüche 1-6, das in den Aminosäureseitenketten chemisch modifiziert ist und/oder bei dem die Aminogruppe des N-terminalen Endes mit elektrophilen Reagentien modifiziert wurde und/oder die Carboxygruppe des C-terminalen Endes des Peptids mit nucleophilen Reagentien modifiziert wurde.

8. Fragmente der Peptide gemäß einem der Ansprüche 1-7, die durch proteolytische Spaltung mit Pröteasen erhältlich sind, wobei die Fragmente fungizide und/oder antibiotische Wirkungen zeigen.

9. Medikament, das wenigstens eines der Peptide gemäß einem der Ansprüche 1-8 in einer wirksamen Menge zur Behandlung von Krankheiten enthält, gegebenenfalls im Gemisch mit weiteren aktiven Verbindungen und/oder Trägermaterialien oder Adjuvantien.

10. Medikament gemäß Anspruch 9, das für die örtliche Verabreichung oder Verabreichung auf Schleimhäuten zubereitet ist.

11. Medikament gemäß Anspruch 9 zur Behandlung von Pilzkrankheiten und/oder zur Behandlung von Krankheiten, die von eukaryontischen Organismen, wie Trichomonaden in der Vagina, oder Organismen wie Filarien oder Plasmodium verursacht werden.

12. Verfahren zur Verwendung eines Peptids gemäß einem der Ansprüche 1-8 als Konservierungsstoff von Lebensmitteln oder verderblichen Waren oder als Adjuvans für Fermentierungsprozesse.

13. Verfahren zur Verwendung eines Peptids gemäß einem der Ansprüche 1-8 zur Herstellung eines Medikaments gemäß den Ansprüchen 9-11.

## Revendications

1. Peptide ayant la séquence d'acides aminés
H₂N-X₁-R-X₂-COOH (formule I)
dans laquelle
X₁ est soit zéro soit
X₁ et/ou X₂ sont un résidu représentant au moins cinq résidus d'acides aminés (symbolisés dans le code des acides aminés à une lettre), de préférence des acides aminés naturels,
étant entendu que X₁ et/ou X₂ contiennent au moins un résidu d'acide aminé basique immédiatement suivi par un résidu d'acide aminé hydrophobe et X₁ et/ou X₂ contiennent au moins un résidu glutamine
étant entendu que
X₁ ne représente pas : KTKLTEEEKNRLNFLKKISQ
X₁ ne représente pas : KNRLNFLKKISQ
X₂ ne représente pas : RYQFALPQYLKTVYQHQK

2. Peptide selon la revendication 1, dans lequel
X₁ représente -Xₘ-K-B-Xₙ- où
X est tout résidu d'acide aminé ayant au moins un Q,
B est tout résidu d'acide aminé hydrophobe ;
m = 0 à 40, n = 0 à 8 et
X₂ est Q.

3. Peptide selon la revendication 1, dans lequel
X₁ est zéro ou représente Q,
X₂ représente -Xₙ-K-B-Xₘ-,
X, B, m et n ayant la même signification que celle définie dans la revendication 2.

4. Peptide selon la revendication 1, dans lequel
X₁ représente -Xₘ-K-B-Xₙ- et
X₂ représente -Xₙ-K-B-Xₘ,
X, B, m et n ont les mêmes significations que celles définies dans la revendication 2.

5. Peptide selon l'une quelconque des revendications 1 à 4, ayant des acides D- et/ou L-aminés.

6. Peptide selon l'une quelconque des revendications 1 à 5, ayant les structures

7. Peptide selon l'une quelconque des revendications 1 à 6, qui est chimiquement modifié dans les chaînes latérales des acides aminés et/ou le groupe amino de l'extrémité N-terminale est modifié avec des réactifs électrophiles et/ou la fraction carboxyle de l'extrémité C-terminale du peptide est modifiée avec des réactifs nucléophiles.

8. Fragments des peptides selon l'une quelconque des revendications 1 à 7, pouvant être obtenus par clivage protéolytique avec des protéases, ces fragments présentant des effets antifongiques et/ou antibiotiques.

9. Médicament comprenant au moins l'un des peptides selon l'une quelconque des revendications 1 à 8 en une quantité efficace pour traiter des maladies, éventuellement en mélange avec d'autres composés actifs et/ou matières véhicules ou adjuvants.

10. Médicament selon la revendication 9, formulé pour l'administration topique ou l'administration sur une muqueuse.

11. Médicament selon la revendication 9 pour traiter des maladies fongiques, et/ou le traitement de maladies provoquées par des organimes eucaryotes tels que des trichomonas dans le vagin, ou des organismes tels qu'une filaire ou un plasmodium.

12. Procédé d'utilisation d'un peptide selon l'une quelconque des revendications 1 à 8 en tant que conservateur pour aliments ou biens périssables ou en tant qu'adjuvant pour des traitements de fermentation.

13. Procédé d'utilisation d'un peptide selon l'une quelconque des revendications 1 à 8 pour la fabrication d'un médicament selon les revendications 9 à 11.


### SEQUENCE LISTING

(1) GENERAL INFORMATION:
   (i) APPLICANT:
      (A) NAME: HaemoPep Pharma GmbH and Prof. Dr. Wolf-Georg Forssmann
      (B) STREET: Feodor-Lynen-Strasse 5
      (C) CITY: Hannover
      (E) COUNTRY: Germany
      (F) POSTAL CODE (ZIP): 30625
   (ii) TITLE OF INVENTION: Antibiotic peptides from bovine milk
   (iii) NUMBER OF SEQUENCES: 5
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
(2) INFORMATION FOR SEQ ID NO: 1:
   (i) SEQUENCE CHARACTERISTICS:
      (A) LENGTH: 39 amino acids
      (B) TYPE: amino acid
      (C) STRANDEDNESS: single
      (D) TOPOLOGY: unknown
   (ii) MOLECULE TYPE: peptide
   (iii) HYPOTHETICAL: NO
   (iv) ANTI-SENSE: NO
   (xi) SEQUENCE DESCRIPTION: SEQ ID NO: 1:
   Lys Thr Lys Leu Thr Glu Glu Glu Lys Asn Arg Leu Asn Phe Leu Lys
   1               5                   10                  15
   Lys Ile Ser Gln Arg Tyr Gln Lys Phe Ala Leu Pro Gln Tyr Leu Lys
               20                  25                  30
   Thr Val Tyr Gln His Gln Lys
               35
(2) INFORMATION FOR SEQ ID NO: 2:
   (i) SEQUENCE CHARACTERISTICS:
      (A) LENGTH: 38 amino acids
      (B) TYPE: amino acid
      (C) STRANDEDNESS: single
      (D) TOPOLOGY: unknown
   (ii) MOLECULE TYPE: peptide
   (iii) HYPOTHETICAL: NO
   (iv) ANTI-SENSE: NO
   (xi) SEQUENCE DESCRIPTION: SEQ ID NO: 2:
   Thr Lys Leu Thr Glu Glu Glu Lys Asn Arg Leu Asn Phe Leu Lys Lys
   1               5                   10                  15
   Ile Ser Gln Arg Tyr Gln Lys Phe Ala Leu Pro Gln Tyr Leu Lys Thr
               20                  25                  30
   Val Tyr Gln His Gln Lys
           35
(2) INFORMATION FOR SEQ ID NO: 3:
   (i) SEQUENCE CHARACTERISTICS:
      (A) LENGTH: 31 amino acids
      (B) TYPE: amino acid
      (C) STRANDEDNESS: single
      (D) TOPOLOGY: unknown
   (ii) MOLECULE TYPE: peptide
   (iii) HYPOTHETICAL: NO
   (iv) ANTI-SENSE: NO
   (xi) SEQUENCE DESCRIPTION: SEQ ID NO: 3:
   Lys Asn Arg Leu Asn Phe Leu Lys Lys Ile Ser Gln Arg Tyr Gln Lys
   1               5                   10                  15
   Phe Ala Leu Pro Gln Tyr Leu Lys Thr Val Tyr Gln His Gln Lys
               20                  25                  30
(2) INFORMATION FOR SEQ ID NO: 4:
   (i) SEQUENCE CHARACTERISTICS:
      (A) LENGTH: 19 amino acids
      (B) TYPE: amino acid
      (C) STRANDEDNESS: single
      (D) TOPOLOGY: unknown
   (ii) MOLECULE TYPE: peptide
   (iii) HYPOTHETICAL: NO
   (iv) ANTI-SENSE: NO
   (xi) SEQUENCE DESCRIPTION: SEQ ID NO: 4:
   Arg Tyr Gln Lys Phe Ala Leu Pro Gln Tyr Leu Lys Thr Val Tyr Gln
   1               5                   10                  15
   His Gln Lys
(2) INFORMATION FOR SEQ ID NO: 5:
   (i) SEQUENCE CHARACTERISTICS:
      (A) LENGTH: 15 amino acids
      (B) TYPE: amino acid
      (C) STRANDEDNESS: single
      (D) TOPOLOGY: unknown
   (ii) MOLECULE TYPE: peptide
   (iii) HYPOTHETICAL: NO
   (iv) ANTI-SENSE: NO
   (xi) SEQUENCE DESCRIPTION: SEQ ID NO: 5:
   Lys Asn Arg Leu Asn Phe Leu Lys Lys Ile Ser Gln Arg Tyr Gln
   1               5                   10                  15